# EUROPEAN PATENT SPECIFICATION

(11) **EP 2 370 525 B1**
(45) Date of publication and mention of the grant of the patent: **25.01.2017**
(21) Application number: 09764419.9
(22) Date of filing: 25.11.2009
(51) Int. Cl.: C09D 5/14, C09D 4/06, A61L 29/08, C09D 5/16, A61L 29/16, B05D 3/02, B05D 3/06

(54) **A SOLVENTLESS ANTIMICROBIAL UV CURABLE COATING COMPOSITIONS**
LÖSUNGSMITTELFREIE ANTIMIKROBIEL UV-HÄRTBARE BESCHICHTUNGSZUSAMMENSETZUNGEN
COMPOSITIONS DE REVÊTEMENT SANS SOLVANT ANTIMICROBIENNES DURCISSABLES AUX UV

(30) Priority: 01.12.2008 US 118988 P; 04.03.2009 US 397760
(43) Date of publication of application: 05.10.2011
(73) Proprietor: Becton, Dickinson and Company, Franklin Lakes, NJ 07417-1880 (US)
(72) Inventor: OU-YANG, David Tien-Tung, Woodbury Minnesota 55125 (US)
(74) Representative: dompatent von Kreisler Selting Werner - Partnerschaft von Patent- und Rechtsanwälten mbB
(86) International application number: PCT/US2009/065941
(87) International publication number: WO 2010/065421

(56) References cited:
- WO-A1-98/58989
- WO-A2-2007/021840
- WO-A2-2007/100776
- WO-A2-2008/132045
- US-A1- 2003 119 932

## Description

### BACKGROUND OF THE INVENTION

The present invention relates to UV-curable antimicrobial coating compositions, UV-curable antimicrobial coatings and antimicrobial coatings obtainable therefrom. The compositions can be used in various medical applications. One of the major challenges of modem medical treatment is control of infection and the spread of microbial organisms.

One area where this challenge is constantly presented is in infusion therapy of various types. Infusion therapy is one of the most common health care procedures. Hospitalized, home care, and other patients receive fluids, pharmaceuticals, and blood products via a vascular access device inserted into the vascular system. Infusion therapy may be used to treat an infection, provide anesthesia or analgesia, provide nutritional support, treat cancerous growths, maintain blood pressure and heart rhythm, or many other clinically significant uses.

Infusion therapy is facilitated by a vascular access device. The vascular access device may access a patient's peripheral or central vasculature. The vascular access device may be indwelling for short term (days), moderate term (weeks), or long term (months to years). The vascular access device may be used for continuous infusion therapy or for intermittent therapy.

A common vascular access device is a plastic catheter that is inserted into a patient's vein. The catheter length may vary from a few centimeters for peripheral access, to many centimeters for central access and may included devices such as peripherally inserted central catheters (PICC). The catheter may be inserted transcutaneously or may be surgically implanted beneath the patient's skin. The catheter, or any other vascular access device attached thereto, may have a single lumen or multiple lumens for infusion of many fluids simultaneously.

The vascular access device commonly includes a Luer adapter to which other medical devices may be attached. For example, an administration set may be attached to a vascular access device at one end and an intravenous (IV) bag at the other. The administration set is a fluid conduit for the continuous infusion of fluids and pharmaceuticals. Commonly, an IV access device is a vascular access device that may be attached to another vascular access device, closes the vascular access device, made with these materials can become distorted over time and/or form microcracks on their surfaces. Another issue with this type of coating is that it takes almost 24 hours for the solvent to be completely heat evaporated. Accordingly, conventional technology has persistent problems with processing, performance, and cost.

Another limitation is the availability of suitable antimicrobial agents for use in such coatings. One of the most commonly used antimicrobial agents used in coating medical device is silver. Silver salts and silver element are well known antimicrobial agents in both the medical surgical industry and general industries. They are usually incorporated into the polymeric bulk material or coated onto the surface of the medical devices by plasma, heat evaporation, electroplating, or by conventional solvent coating technologies. These technologies are tedious, expensive, and not environmentally friendly.

In addition, the performance of silver coated medical devices is mediocre at best. For example, it can take up to eight (8) hours before the silver ion, ionized from the silver salts or silver element, to reach efficacy as an antimicrobial agent. As a result, substantial microbial activity can occur prior to the silver coating even becoming effective. Furthermore, the silver compound or silver element has an unpleasant color, from dark amber to black.

WO 2007/021840 discloses a spacer material which is applied in a thin layer and is prepared from a catalyst paste and a base paste. The catalyst paste comprises a polymerizable monomer, a polymerization initiator, a polymerization inhibitor, filler and an antimicrobial agent. The filler can serve as thickening agent to provide a desired level of viscosity and/or thixotropy to the composition; preferably, in the form of silanated fumed silica. The base paste used to form the spacer material composition comprises a polymerizable monomer, a polymerization accelerator, a polymerization inhibitor, a non-polymerizable plasticizer, and filler. The baste paste preferably contains the polymerizable monomers, urethane dimethacrylate (UDMA) and polyether diurethane methacrylate (PDM). A photoactive agent such as, for example, benzophenone, benzoin, alpha-diketones or their derivatives is added to the base or catalyst paste in order to make the composite material light-curable. made with these materials can become distorted over time and/or form microcracks on their surfaces. Another issue with this type of coating is that it takes almost 24 hours for the solvent to be completely heat evaporated. Accordingly, conventional technology has persistent problems with processing, performance, and cost.

Another limitation is the availability of suitable antimicrobial agents for use in such coatings. One of the most commonly used antimicrobial agents used in coating medical device is silver. Silver salts and silver element are well known antimicrobial agents in both the medical surgical industry and general industries. They are usually incorporated into the polymeric bulk material or coated onto the surface of the medical devices by plasma, heat evaporation, electroplating, or by conventional solvent coating technologies. These technologies are tedious, expensive, and not environmentally friendly.

In addition, the performance of silver coated medical devices is mediocre at best. For example, it can take up to eight (8) hours before the silver ion, ionized from the silver salts or silver element, to reach efficacy as an antimicrobial agent. As a result, substantial microbial activity can occur prior to the silver coating even becoming effective. Furthermore, the silver compound or silver element has an unpleasant color, from dark amber to black.

Accordingly, there is a need in the art for improved compositions for providing antimicrobial capability to medical devices of various types, and particularly devices related to infusion therapy. Specifically, there is a need for an effective antimicrobial coating that can be easily applied to medical devices constructed of polymeric materials and metals. There is also a need for improved methods of applying such antimicrobial coatings to medical devices.

### BRIEF SUMMARY OF THE INVENTION

The present invention has been developed in response to problems and needs in the art that have not yet been fully resolved by currently available antimicrobial compositions and methods. Thus, these compositions and methods are developed to reduce complications, such as the risk and occurrence of CRBSIs, by providing improved antimicrobial compositions and methods for use in conjunction with medical devices.

The present invention relates to ultraviolet (UV) (wavelength of approximately 200 nm to 600 nm)-curable coatings that have antimicrobial properties.

Accordingly, there is a need in the art for improved compositions for providing antimicrobial capability to medical devices of various types, and particularly devices related to infusion therapy. Specifically, there is a need for an effective antimicrobial coating that can be easily applied to medical devices constructed of polymeric materials and metals. There is also a need for improved methods of applying such antimicrobial coatings to medical devices.

### BRIEF SUMMARY OF THE INVENTION

The present invention relates to a solventless antimicrobial ultraviolet (UV) curable coating composition comprising:
a UV curable composition comprising from 10 % to 90 % by weight of an oligomer, from 5 % to 90 % by weight of a momoner, and from 1 % to 10 % by weight of a photoinitiator;
0.1 to 30 parts by weight of a rheology modifier selected from the group consisting of organic clay, castor wax, polyamide wax, and polyurethane; and 0.5 to 50 parts by weight of an antimicrobial agent which is not chemically reacted with the other components of the composition, said parts per weight being based on 100 parts per weight of the UV-curable composition.

Furthermore, the invention relates to a UV-curable antimicrobial coating comprising the UV-curable composition as defined in the claims.

Additionally, the present invention relates to cured antimicrobial coating obtainable from the UV-curable coating as defined above by curing with ultraviolet light.

Preferred embodiments of the invention are apparent from the dependent claims.

The present invention has been developed in response to problems and needs in the art that have not yet been fully resolved by currently available antimicrobial compositions and methods. Thus, these compositions and methods are developed to reduce complications, such as the risk and occurrence of CRBSIs, by providing improved antimicrobial compositions and methods for use in conjunction with medical devices.

The present invention relates to ultraviolet (UV) (wavelength of approximately 200 nm to 600 nm)-curable coatings that have antimicrobial properties. The coatings may be cured by light in the range set forth above, namely from 200 nm to 600 nm. In some embodiments, it may be preferable to cure the composition with light in the range of from 300 nm to 450 nm. These coatings are particularly adaptable for use on medical devices, particularly intravascular access devices like needleless valves. As mentioned above, the medical devices are often comprised of polymeric substrates, especially polycarbonate (PC), polyurethane (PU), polyvinyl chloride (PVC), styrene-butadiene rubber (SBR), and acrylics.

In one aspect of the invention the surfaces of such devices are coated with a UV-curable coating (sometimes hereinafter referred to as "UV coating") which comprises a UV curable composition and additional components incorporated therein such as antimicrobial agents uniformly distributed throughout its matrix. The antimicrobial agents are able to diffuse through the matrix and kill microscopic organisms that come in contact with the coating surface. The antimicrobial agents, which are uniformly distributed in the UV coating matrix, gradually diffuse out of the matrix when the matrix is softened by IV fluids. The antimicrobial agents are then available to kill the microbes that come in contact with the coating surface.

The formulations of this invention are generally comprised of a combination of urethane or polyester-type oligomer with acrylate-type functional groups, acrylate-type monomers, photoinitiators, rheological modifiers, and antimicrobial agents. The nano- or micro- sized particles of the antimicrobial agents are uniformly and permanently distributed throughout the whole coating matrix.

The coatings are solventless and can be sprayed, wiped, dipped or distributed by using other conventional coating methods to coat a substrate's surface. They can then be rapidly cured with ultraviolet light. Curing may be completed in seconds or minutes depending on the formulation and curing conditions. The coatings of the present invention are generally efficacious within minutes instead of hours as with conventional coatings. The coatings also generally have a pleasant light color or an even clear color.

A wide variety of oligomers can be used within the scope of the present invention. It is only necessary that the oligomer be capable of UV curing and of carrying antimicrobial agents of the type described herein. For example, the oligomers can be acrylated aliphatic urethanes, acrylated aromatic urethanes, acrylated polyesters, unsaturated polyesters, acrylated polyethers, acrylated acrylics, and the like, or combinations of the above. The acrylated functional group can be mono-functional, di-functional, tri-functional, tetra-functional, penta-functional, or hexa-functional.

As with the oligomers, a wide range of monomers can be used in the present compositions. Once again, it is only necessary that the overall composition be UV-curable and that the composition be capable of carrying the antimicrobial agents. For example, the monomers can be 2-ethyl hexyl acrylate, isooctyl acrylate, isobornylacrylate, 1,6-hexanediol diacrylate, diethylene glycol diacrylate, triethylene glycol diacrylate, pentaerythritol tetra acrylate, penta erythritol tri acrylate, dimethoxy phenyl acetophenone hexyl methyl acrylate, 1,6-hexanediol methacrylate, and the like, or combinations of these compounds.

In order to allow for UV-curing, the composition should be provided with an adequate and compatible photoinitiator. In certain embodiments of the invention, the photoinitiators can be: 1) single molecule cleavage type, such as benzoin ethers, acetophenones, benzoyl oximes, and acyl phosphine oxide, and 2) hydrogen abstraction type, such as Michler's ketone, thioxanthone, anthroguionone, benzophenone, methyl diethanol amine, 2-N-butoxyethyl-4-(dimethylamino) benzoate, and the like, or combinations of these materials.

According to the invention a rheological modifier is added to the composition. The rheological modifier allows the flow characteristics of the composition to be controlled and modified as desired. The rheological modifier can also aid in the uniform distribution of antimicrobial agent and other materials within the composition. Suitable rheological modifiers may include organic clay, castor wax, polyamide wax, polyurethane, and fumed silica or combinations of these materials.

Various antimicrobial agents may be used in the compositions of the present invention. It is only necessary that the antimicrobial agent be compatible with the other components of the compositions and that it be effective in controlling microbial agents. Specifically, it is required that the antimicrobial agent is not chemically reacted with the other components of the composition. As discussed above, in certain embodiments it is preferred that the antimicrobial agent be capable of moving within the matrix of the composition such that it can be delivered to the site of the microbial agent. Examples of suitable antimicrobial agents within the scope of the present invention include be aldehydes, anilides, biguanides, silver element or its compounds, bis-phenols, and quaternary ammonium compounds and the like or combinations of the above.

The coating composition of the invention is solventless. As mentioned above, many conventional coatings employ harsh solvents such as THF and DMF. The present invention is operated without the use of solvents and, therefore, avoids the difficulties presented by the use of conventional solvents.

The formulations also demonstrate good adhesion to numerous plastic surfaces (such as PC, PU, PVC, acrylics, and SBR). The formulation can be cured with adequate ultraviolet light (wavelength of approximately 200 nm to 600 nm, and in certain embodiments in the range of from 300 nm to 450 nm.

Accordingly, the present invention provides antimicrobial coating compositions which overcome many of the limitations of existing technology. The present invention employs known components which have achieved acceptance for medical use. These components are combined and used easily and efficiently. As set forth above, the compositions of the present invention generally including oligomers, monomers, photoinitiators, rheological modifiers, and suitable antimicrobial agents. The resulting compositions are easily applied to the surfaces of medical devices and quickly cured by UV light.

### DETAILED DESCRIPTION OF THE INVENTION

This detailed description of the invention provides additional description of each of the aspects of the invention summarized above. In one aspect of the invention, an antimicrobial ultra violet (UV)-curable coating is provided. The coating comprising a UV curable composition comprising an oligomer, a monomer, and a photoinitiator which are together capable of forming a UV curable polymer composition. The composition also incudes a rheology
modifier in order to improve the flow characteristics of the composition and uniform distribution of components within the compositions. Finally, incorporated within the UV curable coating compositions is an effective antimicrobial agent.

The UV curable coating compositions are comprised primarily of one or more oligomers and one or more monomers, combined with one or more suitable photoinitiators. In the following discussion, the UV curable coating composition will comprise 100 parts by weight. Materials added to the UV curable coating composition may include rheological modifiers, antimicrobial agents, and other additives. These materials will be defined in parts by weight added to 100 parts by weight of the UV curable coating composition.

The oligomer is generally selected from the group consisting of acrylated aliphatic urethanes, acrylated aromatic urethanes, acrylated polyesters, unsaturated polyesters, acrylated polyethers, acrylated acrylics, and the like, or combinations thereof. The acrylated functional group is selected from the group consisting of mono-functional, di-functional, tri-functional, tetra-functional, penta-functional, and hexa-functional acrylates. Any oligomer which is compatible with the other components of the composition is usable within the scope of the present invention. The oligomer comprises from 10 % to 90 % of the
UV curable composition. In preferred embodiments the oligomer comprises 20 % to 80 % of the UV curable composition. In further preferred embodiments of the invention the oligomer comprises from 30% to 70% of the UV curable composition.

The monomer is selected from the group consisting of 2-ethyl hexyl acrylate, isooctyl acrylate, isobornylacrylate, 1,6-hexanediol diacrylate, diethylene glycol diacrylate, triethylene glycol diacrylate, pentaerythritol tetra acrylate, penta erythritol tri acrylate, dimethoxy phenyl acetophenone hexyl methyl acrylate, 1,6 hexanidiol methacrylate and the like, or combinations of these compounds. Once again any monomer which is compatible with the other components of the composition is usable within the scope of the present invention. The monomer comprises from 5 % to 90 % of the UV curable composition. In preferred embodiments the monomer comprises from 10% to 75% of the UV curable composition. In further preferred embodiments of the invention the monomer comprises from 20% to 60% of the UV curable composition.

The photoinitiator is selected from the group consisting of single molecule cleavage type, such as benzoin ethers, acetophenones, benzoyl oximes, and acyl phosphine oxide, and hydrogen abstraction types consisting of Michler's ketone, thioxanthone, anthroguionone, benzophenone, methyl diethanol amine, and 2-N-butoxyethyl-4-(dimethylamino) benzoate. The photoinitiator will also be selected such that it is compatible with the other components of the composition is usable within the scope of the present invention. The photoinitiator comprises from 1.0% to 10% of the UV curable composition. In preferred embodiments the photoinitiator comprises from 1% to 8.5% of the UV curable composition. In further preferred embodiments of the invention the photoinitiator comprises from 2% to 7% of the UV curable composition.

As mentioned above, certain additional components are added to the UV curable composition. Prominent among these are suitable rheological modifiers and antimicrobial agents. As mentioned above, the amounts of these additional components will be expressed in parts by weight added to 100 parts by weight of UV-curable composition.

The rheological modifier is selected from the group consisting of organic clay, castor wax, polyamide wax, and polyurethane, The rheological modifier generally comprises from 0.1 to 30 parts by weight added to 100 parts by weight of UV curable composition, i.e. the UV curable composition is 100 weight units, while the rheological modifier comprises from 0.1 to 30 parts of additional weight. In preferred embodiments, the rheological modifier comprises from 0.1 to 20 parts by weight compared to 100 parts by weight of the UV curable composition. In further preferred embodiments, the rheological modifier comprises from 0.2 to 10 parts by weight compared to 100 parts by weight of the UV curable composition.

The antimicrobial agent is generally selected from the group consisting of aldehydes, anilides, biguanides, silver, silver compound, bis-phenols, and quaternary ammonium compounds. The antimicrobial agent is generally present in the amount of from 0.5 to 50 parts by weight in compared to 100 parts by weight of the UV curable composition. In preferred embodiments, the antimicrobial agent may be present in the amount of from 0.5 to 30 parts by weight of the composition. In further preferred embodiments, the antimicrobial agent is present in the amount of from 0.5 to 20 parts by weight.

The antimicrobial agent may either dissolve in the UV curable composition or may be uniformly distributed therein. In this manner it is found that sufficient antimicrobial agent can migrate within the composition to contact the location of microbial activity. In any event, the antimicrobial agent does not react chemically with the other components of the compositions.

The UV coating formulations can be urethane or polyester type acrylate such as 7104, 7101, 7124-K, 7105-5K from Electronic Materials Inc. (EMI) (EM Breckenridge, Co.), 1168-M, I-20781 from Dymax Corporation (Torrington, CT.), UV 630 from Permabond Engineering Adhesives (Somerset, NJ). The viscosity of the coating should be less than 10,000 mPa·S (cps), preferable below 5,000 mPa·S (cps), and most preferably between 20 to 1,000 mPa·S (cps).

### EXAMPLES

### Reference Example 1

UV-curable compositions within the scope of the present invention were formulated and their microbial kill rate and zone of inhibition were tested as set forth in Table 1 below. Each of the compositions was essentially identical except for the antimicrobial agent which was varied as set forth below. The composition was comprised of a UV curable composition designated EMI 7104. The UV curable composition was comprised of 30-70% oligomer, 20-60% monomer; 2-7% photoinitiator. Added to 100 parts of the UV curable composition was 2.6 parts fumed silica obtained from Cabot and designated Cabot's MS-55. Also added was 7.2 parts antimicrobial agent. The specific antimicrobial agent was used in the formulation were as follows:
Samples #
   1. Chlorhexidine diacetate
   2. Alexidine
   3. Silver sulfadiazine
   4. Silver acetate
   5. Silver citrate hydrate
   6. Cetrimide
   7. Cetyl pyridinium chloride
   8. Benzalkonium chloride
   9. o-phthalaldehyde
   10. Silver element

Each composition was tested on three (3) microbial agents, namely: *Staphylococcus epidermidis* (gram positive bacteria); *Pseudomonas aeruginosa (gram negative bacteria); and Candida albicans (*yeast or fungi*)*. The results are summarized in Table 1.

**Table 1. The Contact Kill and Zone of Inhibition of UV Coating¹ Formulations**

| **Sample #²** | **Contact Kill (% Kill)** | | | | | | | | | **Zone of Inhibition (mm)** | | |
|---|---|---|---|---|---|---|---|---|---|---|---|---|
| | ***S. epidermidis*³** | | | ***P. aeruginosa*** | | | ***C. albicans*** | | | ***S*. *epider*** | ***P. aerug*** | ***C*. *albica n*** |
| | 1 min | 1 hr | 8 hr | 1 min | 1 hr | 8 hr | 1 min | 1 hr | 8 hr | | | |
| 1 | 74.4 | 100 | ND | 90.6 | 100 | ND | growth | 100 | ND | 22.5 | 13.5 | 23.0 |
| 2 | 79.3 | 100 | ND | 71.4 | 100 | ND | growth | 100 | ND | 13.5 | 0.0 | 13.5 |
| 3 | 0.0 | 35.5 | 100 | 44.3 | 85.5 | 100 | growth | growth | 100 | 14.0 | 13.5 | 18.0 |
| 4 | 4.6 | 32.2 | 100 | 20.0 | 29.8 | 100 | growth | growth | growth | 13.0 | 12.0 | 15.0 |
| 5 | 11.5 | 31.4 | 100 | 37.1 | 36.2 | 100 | growth | growth | 100 | 7.0 | 6.5 | 9.0 |
| 6 | 100 | ND | ND | 100 | ND | ND | 100 | ND | ND | 28.5 | 7.5 | 24.0 |
| 7 | 100 | ND | ND | 100 | ND | ND | 100 | ND | ND | 18.0 | 0.0 | 15.0 |
| 8 | 20.7 | ND | 100 | 100 | ND | ND | growth | 100 | ND | 21.5 | 0.0 | 22.5 |
| 9 | 2.3 | 20.3 | 100 | 7.1 | 0.0 | 100 | growth | growth | 100 | 0.0 | 0.0 | 0.0 |
| 10 | 1.2 | 44.1 | 100 | 24.3 | 46.8 | 100 | growth | growth | growth | 105 | 9.0 | 12.0 |

| | | | | | | | | | | | | |
|---|---|---|---|---|---|---|---|---|---|---|---|---|
| ND - no data in view of 100% kill previously Growth - continued microbial growth | | | | | | | | | | | | |

Each of the compositions was generally effective in killing the bacterial agents. All of the compositions, except that containing silver element, were effective in killing *Candida albicans* with one (1) hour. As set forth in Table 1 is appears that cetyl pyridium chloride and cetrimide were generally more effective than the other antimicrobial agents.

### Reference Example 2

In these examples several antimicrobial agents were incorporated into UV curable coating compositions within the scope of the present invention. Each of the formulations included 100 parts of 7104 UV coating, 2.6 parts of fumed silica [designed M-5], and 5.0 parts of antimicrobial agent. Silver and chlorhexidine were included in the test because they are commonly used antimicrobial agents being used in medical technologies. The results of these tests are set forth in Table 2.

**Table 2. Contact Kill and Zone of inhibition of selective antimicrobial agents in UV formulation (5% agents)**

| Products | Contact Kill (%) | | | Zone of Inhibition | | |
|---|---|---|---|---|---|---|
| 1 Minute (5%) | S. Epi | P. Aeru | C. Albi | S. Epi | P. Aeru | C. Albi |
| Chlorhexidine Diacetate | 37.6 | 0.0 | 39.0 | 21.5 | 13.5 | 19.0 |
| Cetrimide | 72.2 | 96.6 | 87.8 | 30.0 | 0.0 | 23.0 |
| Cetyl Pyridium Chloride | 100.0 | 100.0 | 100.0 | 16.5 | 0.0 | 13.0 |
| Benzalkonium Chloride | 15.8 | 0.0 | 58.5 | 23.5 | 0.0 | 23.5 |
| Silver ^{*2} | -- | -- | -- | -- | -- | -- |
| Chlorhexidine Gluconate ^{*2} | -- | -- | -- | -- | -- | -- |

When 7 parts antimicrobial agent is used the results set forth in Table 3 were obtained.

**Table 3. Contact Kill (%) and Zone of inhibition (mm) of selective antimicrobial agents in UV formulation (7% agents)**

| Products | Contact Kill (%) | | | Zone of Inhibition | | |
|---|---|---|---|---|---|---|
| 1 Minute (7%) | S. Epi | P. Aeru | C. Albi | S. Epi | P. Aeru | C. Albi |
| Chlorhexidine Diacetate | 24.6 | 37.7 | 26.3 | 21.8 | 11.5 | 17.3 |
| Cetrimide | 100 | 100 | 97.3 | 28.5 | "+" | 20.5 |
| Cetyl Pyridium Chloride | 100 | 100 | 100 | 16.3 | 0.0 | 13.0 |
| Benzalkonium Chloride | 100 | 100 | 72.9 | 24.8 | 0.0 | 23.5 |
| Silver | 0.0 | 0.0 | 13.7 | 7.5 | 7.5 | 10.0 |
| Chlorhexidine Gluconate | 0.0 | 0.0 | 2.0 | 13.0 | 0.0 | 0.0 |

### Reference Example 3

In Table 4, the formulation set forth above was prepared using cetyl pyridinium chloride (formulation #1) as the antimicrobial agent. This composition has 100% contact kill within 1 min. The same formulation using chlorhexidine diacetate (formulation #4) as the agent has 100% contact kill within 1 hour for all three types of microorganisms. However, both conventional compositions had 100% contact kill for selected microbes only after about 8 hours (both are using silver compound or silver element as the agent).

**Table 4. Commercial Formulation Analysis**

| Products | Contact Kill (%) | | | Zone of Inhibition (mm) | | |
|---|---|---|---|---|---|---|
| | S. Epi | P. Aeru | C. Albi | S. Epi | P. Aeru | C. Albi |
| Commercial Formulation 1 | | | | | | |
| 1 min. | -- | -- | -- | -- | -- | -- |
| 1 hr. | 0.0 | 9.2 | 11.0 | | | |
| 8 hr. | 100 | 99.7 | 0.0 | | | |

| Commercial Formulation 2 | | | | | | |
|---|---|---|---|---|---|---|
| 1 min. | 0.0 | 0.0 | 13.7 | 7.5 | 7.5 | 10.0 |
| 1 hr. | 0.0 | 100 | 95.2 | | | |
| 8 hr. | 100 | 100 | 89.5 | | | |
| (PC)1 | | | | | | |
| 1 min. | 100 | 100 | 100 | 16.3 | 0.0 | 13.5 |
| 1 hr. | 100 | 100 | 100 | | | |
| 8 hr. | 100 | 100 | 100 | | | |
| (PC)4 | | | | | | |
| 1 min. | 24.6 | 37.7 | 26.3 | 21.8 | 11.5 | 17.3 |
| 1 hr. | 100 | 100 | 100 | | | |
| 8 hr. | 100 | 100 | 100 | | | |

| | | | | | | |
|---|---|---|---|---|---|---|
| (PC) 1: Cetyl pyridinium chloride (PC) 4: Chlorhexidine diacetate as the agent | | | | | | |

### Reference Example 4

Table 5 shows that the four agents identified above can have 100% contact kill within 1 hour and last up to almost 4 days when using S. epidermidis as the microbe. However, conventional silver agent formulations have no 1 hour contact kill at all starting from day 1.

**Table 5. Saline Leach Rate Tests for Selected Antimicrobial Agents (1 hr. contact kill by using Staphylococcus epidermidis as the microbe)**

| | 0 Hr | 24 Hrs | 48 Hrs | 72 Hrs | 94 Hrs |
|---|---|---|---|---|---|
| 1 | 100 | 100 | 100 | 100 | 100 |
| 2 | 100 | 100 | 100 | 100 | 100 |
| 3 | 100 | 100 | 100 | 100 | 100 |
| 4 | 100 | 100 | 100 | 100 | 100 |
| 5 | 0.0 | 1.96 | 0.0 | 0.0 | 0.0 |
| 6 | 100 | 76.5 | 55.4 | 87.8 | 100 |

| | | | | | |
|---|---|---|---|---|---|
| * Note: 1. Cetyl pyridinium chloride 2. Cetrimide 3. Benzalkonium chloride 4. Chlorhexidine diacetate 5. Silver 6. Chlorhexidine gluconate | | | | | |

### Reference Example 5

Saline leach tests were conducted on the compositions described above. As set forth in Table 6 it was observed that chlorhexicine gluconate significantly loses its 1 hour contact kill ability after 48 hours when using P. aeruginosa as the microbe. However, the other agents appear to retain contact kill ability for up to 94 hours.

**Table 6. Saline Leach Rate Tests for Selected Antimicrobial Agents (1 hr. contact kill by using Pseudomonas aeruginosa as the microbe)**

| | 0 Hr | 24 Hrs | 48 Hrs | 72 Hrs | 94 Hrs |
|---|---|---|---|---|---|
| 1 | 100 | 100 | 100 | 100 | 100 |
| 2 | 100 | 100 | 100 | 100 | 100 |
| 3 | 100 | 100 | 100 | 100 | 99.4 |
| 4 | 100 | 100 | 100 | 100 | 100 |
| 5 | 100 | 100 | 100 | 100 | 100 |
| 6 | 100 | 96.0 | 96.5 | 1.2 | 0.0 |

| | | | | | |
|---|---|---|---|---|---|
| * Note: 1. Cetyl pyridinium Chloride 2. Cetrimide 3. Benzalkonium chloride 4. Chlorhexidine diacetate 5. Silver 6. Chlorhexidine gluconate | | | | | |

### Reference Example 6

From the data in Table 7, it is clear that both conventional formulations (silver or chlorhexidine gluconate) significantly lose their efficacy after 24 hours when using *Candida albicans* as the microbe. The top four agents tested herein have 100% efficacy for up to 94 hrs.

**Table 7. Saline Leach Rate Tests for Selected Antimicrobial Agents (1 hr. contact kill by using Candida albicans as the microbe)**

| | 0 Hr | 24 Hrs | 48 Hrs | 72 Hrs | 94 Hrs |
|---|---|---|---|---|---|
| 1 | 100 | 100 | 100 | 100 | 100 |
| 2 | 100 | 100 | 100 | 100 | 100 |
| 3 | 100 | 100 | 100 | 100 | 100 |
| 4 | 100 | 100 | 100 | 100 | 100 |
| 5 | 95.2 | 97.6 | 62.4 | 30.0 | 39.7 |
| 6 | 87.6 | 100 | 29.1 | 25.4 | 12.7 |

| | | | | | |
|---|---|---|---|---|---|
| * Note: 7. Cetyl pyridinium chlorine 8. Cetrimide 9. Benzalkonium chloride 10. Chlorhexidine diacetate 11. Silver 12. Chlorhexidine gluconate | | | | | |

### Reference Example 7

In this example several formulations within the scope of the present invention were made. The UV-curable composition was varied using various proprietary formulations manufactured by EMI. Antimicrobial activity was measured and compared to elongation at break. The data is as follows:

**Table 8. Elongation at Break**

| | *S. epidermidis -* 1 Hour | | | | |
|---|---|---|---|---|---|
| | 0 Hour | 24 Hour | 48 Hour | 72 Hour | 96 Hour |
| 1 | 100 | 100 | 100 | 100 | 100 |
| 11 | 100 | 90.5 | 10.6 | 0 | 0 |
| 31 | 90.7 | 0 | 23.4 | 0 | 0 |
| 41 | 100 | 100 | 100 | 100 | 100 |
| 51 | 100 | 100 | 100 | 100 | 100 |

| | *P. aeruginosa -* 1 Hour | | | | |
|---|---|---|---|---|---|
| | 0 Hour | 24 Hour | 48 Hour | 72 Hour | 96 Hour |
| 1 | 100 | 100 | 99.8 | 100 | 100 |
| 11 | 100 | 100 | 0 | 0 | 0 |
| 31 | 100 | 100 | 0 | 0 | 0 |
| 41 | 100 | 100 | 98.8 | 100 | 100 |
| 51 | 100 | 0 | 0 | 0 | 0 |

| | *C. albicans -* 1 Hour | | | | |
|---|---|---|---|---|---|
| | 0 Hour | 24 Hour | 48 Hour | 72 Hour | 96 Hour |
| 1 | 100 | 100 | 91.7 | 89.7 | 97.1 |
| 11 | 100 | 59.6 | 22.2 | 0 | 0 |
| 31 | 50 | 24.5 | 8.33 | 0 | 0 |
| 41 | 100 | 100 | 97.9 | 95.5 | 99.6 |
| 51 | 100 | 73.9 | 12.5 | 0 | 0 |

## Claims

1. A solventless antimicrobial ultraviolet (UV) curable coating composition comprising:
a UV curable composition comprising from 10 % to 90 % by weight of an oligomer, from 5 % to 90 % by weight of a momoner, and from 1 % to 10 % by weight of a photoinitiator;
0.1 to 30 parts by weight of a rheology modifier selected from the group consisting of organic clay, castor wax, polyamide wax, and polyurethane; and
0.5 to 50 parts by weight of an antimicrobial agent which is not chemically reacted with the other components of the composition, said parts per weight being based on 100 parts per weight of the UV-curable composition.

2. The antimicrobial UV curable coating composition of claim 1, wherein the oligomer is selected from the group consisting of acrylated aliphatic urethanes, acrylated aromatic urethanes, acrylated polyesters, unsaturated polyesters, acrylated polyethers, and acrylated acrylics.

3. The antimicrobial UV curable coating of claim 2, wherein the acrylated functional group is selected from the group consisting of mono-functional, di-functional, tri-functional, tetra-functional, penta-functional, and hexa-functional acrylates.

4. The antimicrobial UV curable coating composition of claim 1, wherein the monomer is selected from the group consisting of 2-ethyl hexyl acrylate, isooctyl acrylate, isobornylacrylate, 1,6-hexanediol diacrylate, diethylene glycol diacrylate, triethylene glycol diacrylate, pentaerythritol tetra acrylate, penta erythritol tri acrylate, dimethoxy phenyl acetophenone hexyl methyl acrylate, and 1,6-hexanediol methacrylate.

5. The antimicrobial UV curable coating composition of claim 1, wherein the photoinitiator is selected from the group consisting of benzoin ethers, acetophenones, benzoyl oximes, acyl phosphine oxide, and Michler's ketone, thioxanthone, anthroguionone, benzophenone, methyl diethanol amine, and 2-N-butoxyethyl-4-(dimethylamino) benzoate.

6. The antimicrobial UV curable coating composition of claim 1, wherein the antimicrobial agent is selected from the group consisting of aldehydes, anilides, biguanides, silver, silver compound, bis-phenols, and quaternary ammonium compounds.

7. The antimicrobial UV coating composition of claim 1, wherein the antimicrobial agent is selected from the group consisting of chlorhexidine diacetate, alexidine, and benzalkonium chloride.

8. The antimicrobial coating composition of claim 1, wherein the antimicrobial agent selected from the group consisting of cetrimide, cetyl pyridinium chloride, chlorhexidine diacetate, alexidine, and benzalkonium chloride.

9. The antimicrobial coating composition of claim 8, wherein the antimicrobial agent is alexidine, or wherein the antimicrobial agent is cetrimide and cetyl pyridinium chloride, or wherein the antimicrobial agent is chlorhexidine diacetate, or wherein the antimicrobial agent is benzalkonium chloride.

10. The antimicrobial UV curable coating composition of claim 1, wherein the composition comprises rheological modifier in the amount of from 0.2 to 20 parts by weight in 100 parts by the weight of the UV-curable composition, or wherein the composition comprises rheological modifier in the amount of from 0.2 to 10 parts by weight in 100 parts by weight of the UV-curable composition.

11. The antimicrobial UV curable coating composition of claim 1, or wherein the composition comprises antimicrobial agent in the amount of from 0.5 to 30 parts by weight in 100 parts by weight of the UV-curable composition, or wherein the composition comprises antimicrobial agent in the amount of from 0.5 to 20 parts by weight in 100 parts by weight of the UV-curable composition.

12. The UV curable coating composition of claim 1 comprising:
a) a UV curable composition, having:
from 10% to 90% by weight oligomer selected from the group consisting of acrylated aliphatic urethanes, acrylated aromatic urethanes, acrylated polyesters, unsaturated polyesters, acrylated polyethers, and acrylated acrylics;
from 5% to 90% by weight monomer selected from the group consisting of 2-ethyl hexyl acrylate, isooctyl acrylate, isobornylacrylate, 1,6-hexanediol diacrylate, diethylene glycol diacrylate, triethylene glycol diacrylate, pentaerythritol tetra acrylate, penta erythritol tri acrylate, dimethoxy phenyl acetophenone hexyl methyl acrylate, and 1,6-hexanediol methacrylate;
b) from 0.1 to 30 parts by weight rheology modifier in 100 parts UV curable composition, the rheology modifier selected from the group consisting of organic clay, castor wax, polyamide wax, and polyurethane;
c) from 0.5 to 50 parts by weight antimicrobial agent in 100 parts UV curable composition, the antimicrobial agent selected from the group consisting of aldehydes, anilides, biguanides, silver, silver compound, bis-phenols, and quaternary ammonium compounds; and
d) from 1 to 10 parts photoinitiator.

13. A UV-curable antimicrobial coating comprising the UV-curable composition as defined in any one of claims 1 to 12.

14. A cured antimicrobial coating obtainable from the UV-curable coating as defined in claim 13 by curing with ultraviolet light.

## Patentansprüche

1. Lösungsmittelfreie antimikrobielle Ultraviolett(UV)-härtbare Beschichtungszusammensetzung, umfassend:
eine UV-härtbare Zusammensetzung, umfassend 10 bis 90 Gew.-% eines Oligomers, 5 bis 90 Gew.-% eines Monomers und 1 bis 10 Gew.-% eines Photoinitiators;
0,1 bis 30 Gewichtsteile eines Rheologiemodifikators, der aus der Gruppe ausgewählt ist, die aus organischem Ton, Ricinuswachs, Polyamidwachs und Polyurethan besteht; und
0,5 bis 50 Gewichtsteile eines antimikrobiellen Mittels, das nicht chemisch mit den anderen Komponenten der Zusammensetzung umgesetzt ist, wobei die Gewichtsteile auf 100 Gewichtsteile der UV-härtbaren Zusammensetzung bezogen sind.

2. Antimikrobielle UV-härtbare Beschichtungszusammensetzung gemäß Anspruch 1, wobei das Oligomer aus der Gruppe ausgewählt ist, die aus acrylierten aliphatischen Urethanen, acrylierten aromatischen Urethanen, acrylierten Polyestern, ungesättigten Polyestern, acrylierten Polyethern und acrylierten Acrylen besteht.

3. Antimikrobielle UV-härtbare Beschichtung gemäß Anspruch 2, wobei die acrylierte funktionelle Gruppe aus der Gruppe ausgewählt ist, die aus monofunktionellen, difunktionellen, trifunktionellen, tetrafunktionellen, pentafunktionellen und hexafunktionellen Acrylaten besteht.

4. Antimikrobielle UV-härtbare Beschichtungszusammensetzung gemäß Anspruch 1, wobei das Monomer aus der Gruppe ausgewählt ist, die aus 2-Ethylhexylacrylat, Isooctylacrylat, Isobornylacrylat, 1,6-Hexandioldiacrylat, Diethylenglycoldiacrylat, Triethylenglycoldiacrylat, Pentaerythrittetraacrylat, Pentaerythrittriacrylat, Dimethoxyphenylacetophenonhexylmethylacrylat und 1,6-Hexandiolmethacrylat besteht.

5. Antimikrobielle UV-härtbare Beschichtungszusammensetzung gemäß Anspruch 1, wobei der Photoinitiator aus der Gruppe ausgewählt ist, die aus Benzoinethern, Acetophenonen, Benzoyloximen, Acylphosphinoxid und Michlers Keton, Thioxanthon, Anthroguionon, Benzophenon, Methyldiethanolamin und 2-N-Butoxy-4-(dimethylamino)benzoat besteht.

6. Antimikrobielle UV-härtbare Beschichtungszusammensetzung gemäß Anspruch 1, wobei das antimikrobielle Mittel aus der Gruppe ausgewählt ist, die aus Aldehyden, Aniliden, Biguaniden, Silber, Silberverbindungen, Bisphenolen und quartären Ammoniumverbindungen besteht.

7. Antimikrobielle UV-Beschichtungszusammensetzung gemäß Anspruch 1, wobei das antimikrobielle Mittel aus der Gruppe ausgewählt ist, die aus Chlorhexidindiacetat, Alexidin und Benzalkoniumchlorid besteht.

8. Antimikrobielle Beschichtungszusammensetzung gemäß Anspruch 1, wobei das antimikrobielle Mittel aus der Gruppe ausgewählt ist, die aus Cetrimid, Cetylpyridiniumchlorid, Chlorhexidindiacetat, Alexidin und Benzalkoniumchlorid besteht.

9. Antimikrobielle Beschichtungszusammensetzung gemäß Anspruch 8, wobei es sich bei dem antimikrobiellen Mittel um Alexidin handelt oder wobei es sich bei dem antimikrobiellen Mittel um Cetrimid und Cetylpyridiniumchlorid handelt oder wobei es sich bei dem antimikrobiellen Mittel um Chlorhexidindiacetat handelt oder wobei es sich bei dem antimikrobiellen Mittel um Benzalkoniumchlorid handelt.

10. Antimikrobielle UV-härtbare Beschichtungszusammensetzung gemäß Anspruch 1, wobei die Zusammensetzung Rheologiemodifikator in einer Menge von 0,2 bis 20 Gewichtsteilen in 100 Gewichtsteilen der UV-härtbaren Zusammensetzung umfasst oder wobei die Zusammensetzung Rheologiemodifikator in einer Menge von 0,2 bis 10 Gewichtsteilen in 100 Gewichtsteilen der UV-härtbaren Zusammensetzung umfasst.

11. Antimikrobielle UV-härtbare Beschichtungszusammensetzung gemäß Anspruch 1, wobei die Zusammensetzung antimikrobielles Mittel in einer Menge von 0,5 bis 30 Gewichtsteilen in 100 Gewichtsteilen der UV-härtbaren Zusammensetzung umfasst oder wobei die Zusammensetzung antimikrobielles Mittel in einer Menge von 0,5 bis 20 Gewichtsteilen in 100 Gewichtsteilen der UV-härtbaren Zusammensetzung umfasst.

12. UV-härtbare Beschichtungszusammensetzung gemäß Anspruch 1, umfassend:
a) eine UV-härtbare Zusammensetzung, mit:
10 bis 90 Gew.-% Oligomer, das aus der Gruppe ausgewählt ist, die aus acrylierten aliphatischen Urethanen, acrylierten aromatischen Urethanen, acrylierten Polyestern, ungesättigten Polyestern, acrylierten Polyethern und acrylierten Acrylen besteht;
5 bis 90 Gew.-% Monomer, das aus der Gruppe ausgewählt ist, die aus 2-Ethylhexylacrylat, Isooctylacrylat, Isobornylacrylat, 1,6-Hexandioldiacrylat, Diethylenglycoldiacrylat, Triethylenglycoldiacrylat, Pentaerythrittetraacrylat, Pentaerythrittriacrylat, Dimethoxyphenylacetophenonhexylmethylacrylat und 1,6-Hexandiolmethacrylat besteht;
b) 0,1 bis 30 Gewichtsteile Rheologiemodifikator in 100 Teilen UVhärtbarer Zusammensetzung, wobei der Rheologiemodifikator aus der Gruppe ausgewählt ist, die aus organischem Ton, Ricinuswachs, Polyamidwachs und Polyurethan besteht;
c) 0,5 bis 50 Gewichtsteile antimikrobielles Mittel in 100 Teilen UVhärtbarer Zusammensetzung, wobei das antimikrobielle Mittel aus der Gruppe ausgewählt ist, die aus Aldehyden, Aniliden, Biguaniden, Silber, Silberverbindungen, Bisphenolen und quartären Ammoniumverbindungen besteht; und
d) 1 bis 10 Teile Photoinitiator.

13. UV-härtbare antimikrobielle Beschichtung, die die UV-härtbare Zusammensetzung gemäß einem der Ansprüche 1 bis 12 umfasst.

14. Gehärtete antimikrobielle Beschichtung, die aus der UV-härtbaren Beschichtung gemäß Anspruch 13 durch Härten mit ultraviolettem Licht erhältlich ist.

## Revendications

1. Composition de revêtement sans solvant antimicrobienne durcissable aux ultraviolets (UV) comprenant :
une composition durcissable aux UV comprenant de 10 % à 90 % en poids d'un oligomère, de 5 % à 90 % en poids d'un monomère, et de 1 % à 10 % en poids d'un photo-initiateur ;
de 0,1 à 30 parties en poids d'un modificateur de rhéologie choisi parmi le groupe constitué de l'argile organique, de la cire de ricin, de la cire polyamide, et du polyuréthane ; et
de 0,5 à 50 parties en poids d'un agent antimicrobien qui n'est pas mis à réagir chimiquement avec d'autres constituants de la composition, lesdites parties en poids étant basées sur 100 parties en poids de la composition durcissable aux UV.

2. Composition de revêtement antimicrobienne durcissable aux UV selon la revendication 1, dans laquelle l'oligomère est choisi parmi le groupe constitué des uréthanes aliphatiques acrylés, des uréthanes aromatiques acrylés, des polyesters acrylés, des polyesters insaturés, des polyéthers acrylés, et des acryliques acrylés.

3. Revêtement antimicrobien durcissable aux UV selon la revendication 2, dans lequel le groupe fonctionnel acrylé est choisi parmi le groupe constitué des acrylates monofonctionnels, bifonctionnels, trifonctionnels, tétrafonctionnels, pentafonctionnels, et hexafonctionnels.

4. Composition de revêtement antimicrobienne durcissable aux UV selon la revendication 1, dans laquelle le monomère est choisi parmi le groupe constitué du 2-éthylhexyl-acrylate, de l'acrylate isooctylique, de l'isobornylacrylate, du 1,6-hexanediol diacrylate, du diacrylate de diéthylène glycol, du diacrylate de triéthylène glycol, du tétra-acrylate de pentaérythritol, du triacrylate de pentaérythritol, de acrylate d'hexylméthyle de diméthoxy phénylacétophénone, et du méthacrylate de 1,6-hexanediol.

5. Composition de revêtement antimicrobienne durcissable aux UV selon la revendication 1, dans laquelle le photo-initiateur est choisi parmi le groupe constitué des éthers de benzoïne, des acétophénones, des oximes de benzoyle, de l'oxyde d'acylphosphine, et la cétone de Michler, de la thioxanthone, de l'anthroguinone, de la benzophénone, de la méthyldiéthanolamine, et du 2-N-butoxyéthyl-4-(diméthylamino) benzoate.

6. Composition de revêtement antimicrobienne durcissable aux UV selon la revendication 1, dans laquelle l'agent antimicrobien est choisi parmi le groupe constitué des aldéhydes, des anilides, des biguanides, de l'argent, d'un composé argent, des bisphénols, et des composés d'ammonium quaternaires.

7. Composition de revêtement antimicrobienne durcissable aux UV selon la revendication 1, dans laquelle l'agent antimicrobien est choisi parmi le groupe constitué du diacétate de chlorhexidine, de l'alexidine et du chlorure de benzalkonium.

8. Composition de revêtement antimicrobienne selon la revendication 1, dans laquelle l'agent antimicrobien est choisi parmi le groupe constitué du cétrimide, du chlorure de cétyl pyridinium, du diacétate de chlorhexidine, de l'alexidine, et du chlorure de benzalkonium.

9. Composition de revêtement antimicrobienne selon la revendication 8, dans laquelle l'agent antimicrobien est l'alexidine, ou dans laquelle l'agent antimicrobien est le cétrimide et le cholrure de cétyl pyridinium, ou dans laquelle l'agent antimicrobien est le diacétate de chlorhexidine, ou dans laquelle l'agent antimicrobien est le chlorure de benzalkonium.

10. Composition de revêtement antimicrobienne durcissable aux UV selon la revendication 1, dans laquelle la composition comprend un modificateur de rhéologies en une quantité allant de 0,2 à 20 parties en poids dans 100 parties en poids de la composition durcissable aux UV, ou dans laquelle la composition comprend un modificateur de rhéologie en une quantité allant de 0,2 à 10 parties en poids dans 100 parties en poids de la composition durcissable aux UV.

11. Composition de revêtement antimicrobienne durcissable aux UV selon la revendication 1, ou dans laquelle la composition comprend un agent antimicrobien en une quantité allant de 0,5 à 30 parties en poids dans 100 parties en poids de la composition durcissable aux UV, ou dans laquelle la composition comprend un agent antimicrobien en une quantité allant de 0,5 à 20 parties en poids dans 100 parties en poids de la composition durcissable aux UV.

12. Composition de revêtement durcissable aux UV selon la revendication 1, comprenant :
a) une composition durcissable aux UV, ayant :
de 10 % à 90 % en poids d'un oligomère choisi parmi le groupe constitué des uréthanes aliphatiques acrylés, des uréthanes aromatiques acrylés, des polyesters acrylés, des polyesters insaturés, des polyéthers acrylés, et des acryliques acrylés ;
de 5 % à 90 % en poids d'un monomère choisi parmi le groupe constitué du 2-éthylhexyl-acrylate, de l'acrylate isooctylique, de l'isobornylacrylate, du 1,6-hexanediol diacrylate, du diacrylate de diéthylène glycol, du diacrylate de triéthylène glycol, du tétra-acrylate de pentaérythritol, du triacrylate de pentaérythritol, de l'acrylate d'hexylméthyle de diméthoxy phénylacétophénone, et du méthacrylate de 1,6-hexanediol ;
b) de 0,1 à 30 parties en poids d'un modificateur de rhéologie dans 100 parties en poids de la composition durcissable aux UV, le modificateur de rhéologie étant choisi parmi le groupe constitué de l'argile organique, de la cire de ricin, de la cire polyamide, et du polyuréthane ;
c) de 0,5 à 50 parties en poids d'un agent antimicrobien dans 100 parties en poids de la composition durcissable aux UV, l'agent antimicrobien étant choisi parmi le groupe constitué des aldéhydes, des anilides, des biguanides, de l'argent, d'un composé argent, des bisphénols, et des composés d'ammonium quaternaires ; et
d) de 1 à 10 parties d'un photo-initiateur.

13. Revêtement antimicrobien durcissable aux UV, comprenant la composition durcissable aux UV selon l'une quelconque des revendications 1 à 12.

14. Revêtement antimicrobien durci pouvant être obtenu à partir du revêtement durcissable aux UV selon la revendication 13 par durcissement avec une lumière ultraviolette.
